# NEUE EUROPÄISCHE PATENTSCHRIFT

(11) **EP 1 503 720 B2**
(45) Veröffentlichungstag und Bekanntmachung des Hinweises auf die Entscheidung über den Einspruch: **28.10.2009**
(45) Hinweis auf die Patenterteilung: 10.01.2007
(21) Anmeldenummer: 03747436.8
(22) Anmeldetag: 30.04.2003
(51) Int. Cl.: A61K 8/02, D04H 1/46, A47K 7/03, A61Q 19/10

(54) **HAUTREINIGUNGSTUCH**
SKIN CLEANSING TOWELETTE
LINGETTE POUR LE NETTOYAGE DE LA PEAU

(30) Priorität: 02.05.2002 DE 10219638
(43) Veröffentlichungstag der Anmeldung: 09.02.2005
(73) Patentinhaber: Beiersdorf AG, 20245 Hamburg (DE)
(72) Erfinder: DRUCKS, Anja, 22399 Hamburg (DE); GRÖNWOLDT, Karen, 22607 Hamburg (DE); SCHULZIG, Mark, 20249 Hamburg (DE); MAX, Heiner, 22529 Hamburg (DE)
(74) Vertreter: Rabanus, Birgit
(86) Internationale Anmeldenummer: PCT/EP2003/004486
(87) Internationale Veröffentlichungsnummer: WO 2003/092635

(56) Entgegenhaltungen:
- WO-A-01/02479
- WO-A-01/35906
- WO-A-01/79418
- US-A- 5 714 445

## Beschreibung

Die vorliegende Erfindung betrifft ein Hautreinigungstuch mit einem Vlies-Träger und einem Tränkungsmedium, sowie seine Verwendung und Herstellung. Solche Hautreinigungstücher sind aus WO01/79418 bekannt.

Der Wunsch nach sauberer Haut ist wohl so alt wie die Menschheit, denn Schmutz, Schweiß und Reste abgestorbener Hautpartikel bieten den idealen Nährboden für Krankheitserreger und Parasiten aller Art. Die Lust an der Körperhygiene wurde stetig verstärkt, als in den 60er Jahren des 20. Jahrhunderts neben der "klassischen" Seife auch flüssige Reinigungsmittel mit neuentwickelten synthetischen Tensiden formuliert werden konnten. Baden und Duschen sind seitdem aus unserem täglichen Leben nicht mehr wegzudenken und den Verbrauchern stehen heutzutage eine Vielzahl von Produkten für die Reinigung der verschiedenen Körperpartien zur Verfügung.

Eine besondere Gruppe an Hautreinigungsprodukten bilden dabei die Gesichtsreinigungsprodukte. Da die Gesichtshaut besonders empfindlich ist, und das Gesicht das am stärksten wahrgenommenes Körperteil und "Aushängeschild" des Menschen ist, werden für die Gesichtsreinigung besonders milde und die Haut nicht reizende Produkte eingesetzt.

Im Bereich der dekorativen Kosmetik finden eine Vielzahl von unterschiedlichen Stoffen Anwendung. Als Farbstoffe werden neben anorganischen Pigmenten wie Silikaten [Magnesiumsilikat (Talkum), Aluminiumsilikat (Kaolin)] und Metalloxiden (Chrom-, Eisen-, Mangan-, Titan- und Zinkoxiden) organische Farbpigmente eingesetzt. Als Bindemittel finden unter anderem Stearinsäureester, Lanolinalkohol und -acetat Verwendung. In vielen Formulierungen werden Wachse wie Bienenwachs oder Carnaubawachs und Öle wie Paraffinöle, Silikonöle oder Ricinusöl eingesetzt. Des weiteren können dekorative Kosmetika Konservierungsstoffe, Antioxidantien, Verdickungsmittel und andere Zusätze enthalten.

Um diese Vielzahl völlig unterschiedlicher Stoffe von der Haut zu entfemen, bedarf es entsprechender kosmetischer Reinigungsmittel. Sie müssen unpolare Verbindungen wie Wachse, Öle und Silikonverbindungen lösen und gleichzeitig die schwerlöslichen Pigmente wie Talkum oder Titandioxid aufnehmen. Dies gilt insbesondere für Wimperntusche, Mascara, Lidschatten und Kajalstifte. Andererseits müssen sie so hautverträglich wie möglich sein, um bei den Anwendern keine Hautrötungen oder Schleimhautirritationen auszulösen.

Eine besondere Ausführungsform kosmetischer Reinigungsmittel stellen die Tücher dar. Diese können mit einer Reinigungszubereitung getränkt sein, welche die mechanische Entfernung des Schmutzes von der Haut unterstützt. Kommerziell erhältliche mit Reinigungsmitteln getränkte Tücher haben darüber hinaus den Vorteil, dass in ihnen die Reinigungszubereitung bereits in der richtigen Menge vorgegeben ist. Außerdem vermeiden sie die Nachteile von in Flaschen aufbewahrten Reinigungsmitteln, deren Verpackung zerbrechen und deren Inhalt "auslaufen" kann. Ein weiterer Nachteil von Flaschen gegenüber Tüchern besteht in dem Umstand, dass deren Inhalt bei der Entnahme durch den Kontakt mit Hand und Fingern mit Mikroorganismen kontaminiert wird. Ein weiterer Vorteil von Tüchern ist es auch, dass sie sich bequem, bruchsicher und in abgezählter Menge mit auf Reisen nehmen lassen.

Mit den derzeit im Handel erhältlichen Hautreinigungstüchern ist jedoch keine befriedigende Reinigung der Haut möglich, insbesondere nicht im Gesicht, so dass sich Verschmutzungen auf der Hautoberfläche und insbesondere wasserfestes Make-up nicht ausreichend intensiv und sanft entfernen lassen.

Auch im Stand der Technik wurden bereits derartige Hautreinigungstücher beschrieben. So verweist WO 99/25318 auf vorbekannte Hautreinigungstücher mit Löchern bzw. Öffnungen, die als nachteilig angesehen wurden und gemäß dieser Druckschrift dahingehend verbessert wurden, dass statt dessen auf der Basis-Oberfläche erhabene Muster vorgesehen wurden, womit sich eine dreidimensionale Prägung der Oberfläche ergibt. Dies erfordert eine aufwendige Herstellung und führt zu keiner ausreichend sanften Hautreinigung.

Auch aus US 6.280.757 sind Hautreinigungstücher bekannt, die sich durch Löcher in seinem Träger auszeichnen, jedoch von ihrem gesamten Aufbau her bei geringer Festigkeit und einem rauen Hautgefühl nicht überzeugen können.

Es war daher die Aufgabe der vorliegenden Erfindung, die Mängel des Standes der Technik zu beseitigen und Hautreinigungstücher zu entwickeln, die hautfreundlich und zugleich reißfest sind, und die hautschonend sanft und hautverträglich insbesondere im Gesicht angewendet werden können.

Überraschend gelöst wird die Aufgabe durch Hautreinigungstücher, wie sie näher in den Ansprüchen gekennzeichnet sind.

So zeichnen sich die erfindungsgemäßen Hautreinigungstücher aus durch einen Vlies-Träger, der mit einem Tränkungsmedium getränkt ist, wobei beide Oberflächen (Seiten) des Vlies-Trägers eben ausgebildet sind, der Vlies-Träger mit Löchern versehen und wasserstrahlverfestigt ist, und das Tränkungsmedium ein öliges, emulsionsartiges oder tensidhaltiges Tränkungsmedium ist.

Vorzugsweise hat der Vlies-Träger ein Gewicht von 35 - 120 g/m² und ist 0,4 - 2 mm dick ist, die Löcher sind gestanzt, und der Vlies-Träger ist einlagig ausgebildet.

Weiterhin vorzugsweise ist der Vlies-Träger auf einer ebenen Oberfläche hergestellt, die Anzahl der Löcher pro Quadratzentimeter ist größer/gleich 15, das Tränkungsmedium macht 100 - 400 Gew.% bezogen auf das Gewicht des Vlies-Trägers aus, und die Fasern des Vlies-Trägers weisen eine Wasseraufnahmerate von mehr als 60 mm/(10 min) und/oder ein Wasseraufnahmevermögen von mehr als 5 g/g auf.

Die anspruchsgemäße, beidseitig ebene Ausgestaltung in Kombination mit Löchern und Wasserstrahlverfestigung führt zu Eigenschaften, die das erfindungsgemäße Hautreinigungstuch überaus geeignet machen zur sanften Hautreinigung, insbesondere im Gesicht.

Besonders geeignet ist das erfindungsgemäße Hautreinigungstuch zur hautschonenden, sanften und verträglichen Hautreinigung, insbesondere zur Gesichtsreinigung, besonders zur Entfernung von wasserfestem Make-up.

Damit zeichnen sich die erfindungsgemäßen Hautreinigungstücher durch eine glückliche Kombination von Merkmalen aus, die in ihrer Gesamtheit zu einem hautfreundlichen, sanft anmutenden Produkt führen, wobei sich auch die Reißfestigkeit aufgrund der Verfestigung des Vlies-Trägers als für die praktische Anwendung ausreichend erwiesen hat. Auch die Zusammensetzung der Reinigungszubereitung bzw. des Tränkungsmediums kann besonders vielseitig gestaltet werden.

Zwar sind aus EP 759.291 weitere Hautreinigungstücher bekannt, die spezielle Micro-Emulsionen enthalten, die jedoch keinen Hinweis auf die erfindungsgemäßen Hautreinigungstücher liefern.

Es hat sich als vorteilhaft herausgestellt für den Vlies-Träger, wenn dieser ein Gewicht von 35 bis 120 g/m², vorzugsweise von 40 bis 60 g/m², hat (gemessen bei 20 °C ± 2 °C und bei einer Feuchtigkeit der Raumluft von 65 % ± 5 % für 24 Stunden).

Die Dicke des Vlies-Trägers beträgt vorzugsweise 0,4 mm bis 2 mm, insbesondere 0,6 mm bis 0,9 mm.

Als Ausgangsmaterialien für den Vliesstoff des Trägers können generell alle organischen und anorganischen Faserstoffe auf natürlicher und synthetischer Basis verwendet werden. Beispielhaft seien Viskose, Baumwolle, Zellulose, Jute, Hanf, Sisal, Seide, Wolle, Polypropylen, Polyester, Polyethylenterephthalat (PET), Aramid, Nylon, Polyvinylderivate, Polyurethane, Polylactid, Polyhydroxyalkanoat, Celluloseester und/oder Polyethylen sowie auch mineralische Fasern wie Glasfasern oder Kohlenstoffasern angeführt. Die vorliegende Erfindung ist aber nicht auf die genannten Materialien beschränkt, sondern es können eine Vielzahl weiterer Fasern zur Vliesbildung eingesetzt werden. Es ist insbesondere vorteilhaft im Sinne der vorliegenden Erfindung, wenn die eingesetzten Fasern nicht wasserlöslich sind.

In einer besonders vorteilhaften Ausführungsform des Vlieses bestehen die Fasern aus einer Mischung aus 70 % Viskose und 30 % PET.

Besonders vorteilhaft sind auch Fasern aus hochfesten Polymeren wie Polyamid, Polyester und/oder hochgerecktem Polyethylen.

Darüber hinaus können die Fasern auch eingefärbt sein, um die optische Attraktivität des Vlieses betonen und/oder erhöhen zu können. Die Fasern können zusätzlich UV-Stabilsatoren und/oder Konservierungsmittel enthalten.

Die zur Bildung des Vlieses eingesetzten Fasern weisen eine Wasseraufnahmerate von mehr als 60 mm/[10 min] (gemessen mit dem EDANA Test 10.1-72), insbesondere mehr als 80 mm/[10 min] auf.

Ferner weisen die zur Bildung des Vlieses eingesetzten Fasern vorzugsweise ein Wasseraufnahmevermögen von mehr als 5 g/g (gemessen mit dem EDANA Test 10.1-72), insbesondere mehr als 8 g/g auf.

Die Löcher haben vorzugsweise einen Durchmesser von höchstens 0,49 mm, und ihre Anzahl pro Quadratzentimeter ist insbesondere größer/gleich 15.

Die erfindungsgemäßen Hautreinigungstücher zeichnen sich dadurch aus, dass sie sowohl für ölige als auch emulsionsartige oder tensidhaltige Tränkungsmedien aufnehmen können, wobei hierzu insbesondere auf die bekannten derartigen Tränkungsmedien zurück gegriffen werden kann.

Tensidhaltige Tränkungsmedien enthalten vorteilhaft anionische Tenside, kationische Tenside, amphotere Tenside, nichtionische Tenside und/oder Polysorbate.

Die Tränkungsmedien enthalten vorteilhaft ein oder mehrere waschaktive anionische, kationische, amphotere und/oder nicht-ionische Tenside. Es ist besonders vorteilhaft das oder die erfindungsgemäß eingesetzten waschaktiven Tenside aus der Gruppe der Tenside zu wählen, welche einen HLB-Wert von mehr als 15 haben, ganz besonders vorteilhaft sind solche, welchen einen HLB-Wert von mehr als 25 haben.

Besonders vorteilhafte waschaktive anionische Tenside im Sinne der vorliegenden Erfindung sind beispielsweise
Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroylsarcosinat und Natriumcocoylsarkosinat,

Sulfonsäuren und deren Salze, wie
■ Acyl-isethionate, z.B. Natrium-/ Ammoniumcocoyl-isethionat,
■ Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie Schwefelsäureester, wie
■ Alkylethersulfat, beispielsweise Natrium-, Ammonium-, Magnesium-, MIPA-, TIPA- Laurethsulfat, Natriummyrethsulfat und Natrium C₁₂₋₁₃ Parethsulfat,
■ Alkylsulfate, beispielsweise Natrium-, Ammonium- und TEA- Laurylsulfat.

Besonders vorteilhafte waschaktive kationische Tenside im Sinne der vorliegenden Erfindung sind quarternäre Tenside. Quaternäre Tenside enthalten mindestens ein N-Atom, das mit 4 Alkyl- oder Arylgruppen kovalent verbunden ist. Vorteilhaft sind Benzalkoniumchlorid, Alkylbetain, Alkylamidopropylbetain und Alkyl-amidopropylhydroxysultain.

Besonders vorteilhafte waschaktive amphotere Tenside im Sinne der vorliegenden Erfindung sind beispielsweise
■ Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,

Besonders vorteilhafte waschaktive nicht-ionische Tenside im Sinne der vorliegenden Erfindung sind
■ Alkanolamide, wie Cocamide MEA/ DEA/ MIPA,
■ Ester, die durch Veresterung von Carbonsäuren mit Ethylenoxid, Glycerin, Sorbitan oder anderen Alkoholen entstehen,
■ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Weitere vorteilhafte anionische Tenside sind
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat,
■ Phosphorsäureester und Salze, wie beispielsweise DEA-Oleth-10 Phosphat und Dilaureth-4 Phosphat,
■ Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat.

Weitere vorteilhafte amphotere Tenside sind
■ N-Alkylaminosäuren, beispielsweise Aminopropylalkylglutamid, Alkylaminopropionsäure, Natriumalkylimidodipropionat und Lauroamphocarboxyglycinat.

Weitere vorteilhafte nicht-ionische Tenside sind Alkohole.

Weitere geeignete anionische Tenside im Sinne der vorliegenden Erfindung sind ferner
■ Acylglutamate wie Di-TEA-palmitoylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen sowie Carbonsäuren und Derivate, wie
■ beispielsweise Laurinsäure, Aluminiumstearat, Magnesiumalkanolat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.

Weitere geeignete kationische Tenside im Sinne der vorliegenden Erfindung sind ferner
■ Alkylamine,
■ Alkylimidazole und
■ ethoxylierte Amine.

Weitere geeignete nicht-ionische Tenside im Sinne der vorliegenden Erfindung sind ferner Aminoxide, wie Cocoamidopropylaminoxid.

Es ist vorteilhaft im Sinn der vorliegenden Erfindung, wenn der Gehalt an einem oder mehreren waschaktiven Tensiden in der kosmetischen oder dermatologischen Reinigungszubereitung bzw. dem Tränkungsmedium aus dem Bereich von 5 bis 25 Gew.-%, ganz besonders vorteilhaft von 10 bis 20 Gew.-% gewählt wird, jeweils bezogen auf das Gesamtgewicht der Zubereitungen.

Polysorbate stellen eine Verbindungsklasse dar, die sich vom Sorbitan, einem aus Sorbit durch Abspaltung zweier Äquivalente Wasser gewonnenem Furanderivat, ableiteten. Die Hydroxylgruppen des Sorbitans sind mit Polyethylenglykolen verethert, deren Enden mit Fettsäuren verestert sein können. Sie lassen sich allgemein durch die Formel 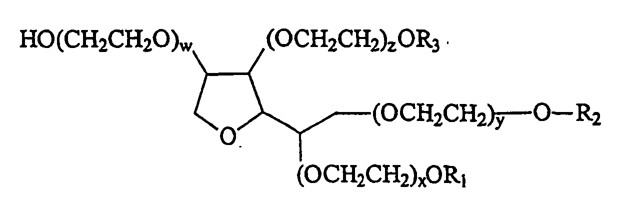 R₁, R₂, R₃ = H, Fettsäurerest darstellen. Im Sinne der Erfindung vorteilhafte Polysorbate sind dabei das
- Polyoxyethylen(20)sorbitanmonolaurat (Tween 20, CAS-Nr.9005-64-5)
- Polyoxyethylen(4)sorbitanmonolaurat (Tween 21, CAS-Nr.9005-64-5)
- Polyoxyethylen(4)sorbitanmonostearat (Tween 61, CAS-Nr. 9005-67-8)
- Polyoxyethylen(20)sorbitantristearat (Tween 65, CAS-Nr. 9005-71-4)
- Polyoxyethylen(20)sorbitanmonooleat (Tween 80, CAS-Nr. 9005-65-6)
- Polyoxyethylen(5)sorbitanmonooleat (Tween 81, CAS-Nr. 9005-65-5)
- Polyoxyethylen(20)sorbitantrioleat (Tween 85, CAS-Nr. 9005-70-3).

Ganz besonders vorteilhaft sind insbesondere
- Polyoxyethylen(20)sorbitanmonopalmitat (Tween 40, CAS-Nr. 9005-66-7)
- Polyoxyethylen(20)sorbitanmonostearat (Tween 60, CAS-Nr. 9005-67-8).

Diese werden, erfindungsgemäß vorteilhaft in einer Konzentration von 0,1 bis 5 Gewichts-% und insbesondere in einer Konzentration von 1,5 bis 2,5 Gewichts-%, bezogen auf das Gesamtgewicht der Formulierung einzeln oder als Mischung mehrer Polysorbate, eingesetzt.

Erfindungsgemäß besonders vorteilhaft sind insbesondere Tränkungsmedien mit jeweils 1 Gewichts-% Polyoxyethylen(20)sorbitanmonostearat (Tween 60) und 1 Gewichts-% Polyoxyethylen(20)sorbitanmonopalmitat (Tween 40).

Erfindungsgemäß vorteilhafte Polyacrylate sind Polymere der Acrylsäure, insbesondere solche, die aus der Gruppe der sogenannten Carbomere oder Carbopole (Carbopol® ist eigentlich eine eingetragene Marke der B. F. Goodrich Company) gewählt werden. Polyacrylate sind Verbindungen der allgemeinen Strukturformel 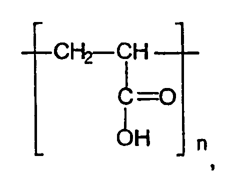 deren Molgewicht zwischen ca. 400 000 und mehr als 4 000 000 betragen kann. In die Gruppe der Polyacrylate gehören ferner Acrylat-Alkylacrylat-Copolymere, beispielsweise solche, die sich durch die folgende Struktur auszeichnen:

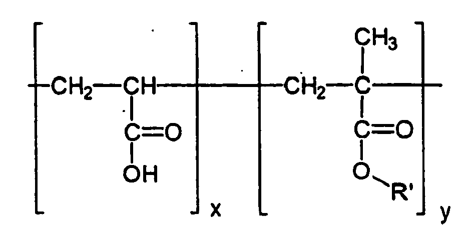

Darin stellen R' einen langkettigen Alkylrest und x und y Zahlen dar, welche den jeweiligen stöchiometrischen Anteil der jeweiligen Comonomere symbolisieren. Auch diese Polyacrylate sind vorteilhaft im Sinne der vorliegenden Erfindung.

Vorteilhafte Carbopole sind beispielsweise die Typen 907, 910, 934, 940, 941, 951, 954, 980, 981, 1342, 1382, 2984 und 5984 oder auch die Typen ETD (Easyto-disperse) 2001, 2020, 2050, wobei diese Verbindungen einzeln oder in beliebigen Kombinationen untereinander vorliegen können.

Besonders bevorzugt sind Carbopol 981, 1382 und ETD 2020 (sowohl einzeln als auch in Kombination).

Ferner vorteilhaft im Sinne der vorliegenden Erfindung sind die den Acrylat-Alkylacrylat-Copolymeren vergleichbaren Copolymere aus C₁₀₋₃₀-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind die unter den Handelsbezeichnungen Pemulen TR1 und Pemulen TR2 bei der B. F. Goodrich Company erhältlichen.

Es ist vorteilhaft im Sinn der vorliegenden Erfindung, wenn der Gehalt an einem oder mehreren Polyacrylaten in dem Tränkungsmedium aus dem Bereich von 0,5 bis 2 Gew.-%, ganz besonders vorteilhaft von 0,7 bis 1,5 Gew.-% gewählt wird, jeweils bezogen auf das Gesamtgewicht der Zubereitung.

Gemäß einer besonders bevorzugten Ausführungsform werden Tränkungsmedien eingesetzt, welche als waschaktive Tenside mindestens ein anionisches Tensid und mindestens einen Verdicker auf Basis von C₁₀-C₃₀-Alkylacrylaten als Polyacrylat enthalten. Natriumlaurethsulfat ist dabei als anionisches Tensid besonders bevorzugt. Diese Kombination an Inhaltsstoffen zeichnet sich durch ihre Stabilität, ihr Schaumbildungsverhalten sowie durch ihr besonders angenehmes Hautgefühl aus.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Antioxidantien, Konservierungsmittel, Bakterizide, Pigmente, die eine färbende Wirkung haben, Verdickungsmittel, weichmachende, anfeuchtende und/oder feuchthaltende Substanzen, oder andere übliche Bestandteile einer kosmetischen oder dermatologischen Formulierung wie Alkohole, Polyole, Polyether, Glycerin, Glykole, Polymere, Schaumstabilisatoren; Elektrolyte, organische Lösemittel oder Silikonderivate.

Die Herstellung eines erfindungsgemäßen Hautreinigungstuches kann vorteilhaft so durchgeführt werden, dass der Vlies-Träger auf einer ebenen Oberfläche so aufgebaut und wasserstrahlverfestigt wird, dass seine beiden Seiten eben ausgebildet werden, worauf in den so hergestellten Vlies-Träger Löcher gestanzt werden, der Vlies-Träger mit dem Tränkungsmedium getränkt wird und das Produkt gegebenenfalls zu praxisgerechter Größe geschnitten und verpackt wird.

## Patentansprüche

1. Hautreinigungstuch mit einem Vlies-Träger, der mit einem Tränkungsmedium getränkt ist, wobei
beide Oberflächen (Seiten) des Vlies-Trägers eben ausgebildet sind,
der Vlies-Träger mit Löchern versehen und wasserstrahlverfestigt ist,
das Tränkungsmedium ein öliges, emulsionsartiges oder tensidhaltiges Tränkungsmedium ist, **dadurch gekennzeichnet, dass**
die Anzahl der Löcher pro Quadratzentimeter größer/gleich 15 ist,
die Fasern des Vlies-Trägers ein Wasseraufnahmevermögen von mehr als 5 g/g aufweisen und die Fasern des Vlies-Trägers eine Wasseraufnahmerate von mehr als 60 mm/(10 min) aufweisen.

2. Hautreinigungstuch nach Anspruch 1, **dadurch gekennzeichnet, dass** der Vlies-Träger ein Gewicht von 35 - 120 g/m² hat.

3. Hautreinigungstuch nach einem der Ansprüche 1 oder 2, **dadurch gekennzeichnet, dass** der Vlies-Träger 0,4 - 2 mm dick ist.

4. Hautreinigungstuch nach einem der Ansprüche 1 - 3, **dadurch gekennzeichnet, dass** die Löcher in den Vlies-Träger gestanzt sind.

5. Hautreinigungstuch nach einem der Ansprüche 1 - 4, **dadurch gekennzeichnet, dass** der Vlies-Träger einlagig ausgebildet ist.

6. Hautreinigungstuch nach einem der Ansprüche 1 - 5, **dadurch gekennzeichnet, dass** der Vlies-Träger auf einer ebenen Oberfläche hergestellt ist.

7. Hautreinigungstuch nach einem der Ansprüche 1 - 6, **dadurch gekennzeichnet, dass** das Tränkungsmedium 100 - 400 Gew.% bezogen auf das Gewicht des Vlies-Trägers ausmacht.

8. Verwendung eines Hautreinigungstuches nach einem der Ansprüche 1 - 7 zur hautschonenden, sanften und verträglichen Hautreinigung, insbesondere zur Gesichtsreinigung, besonders zur Entfernung von wasserfestem Make-up.

9. Verfahren zur Herstellung eines Hautreinigungstuches gemäß einem der Ansprüche 1 - 7, **dadurch gekennzeichnet, dass** der Vlies-Träger auf einer ebenen Oberfläche so aufgebaut und wasserstrahlverfestigt wird, dass seine beiden Seiten eben ausgebildet werden, worauf in den so hergestellten Vlies-Träger Löcher gestanzt werden, der Vlies-Träger mit dem Tränkungsmedium getränkt wird und das Produkt gegebenenfalls zu praxisgerechter Größe geschnitten und verpackt wird.

## Claims

1. Skin cleansing towelette with a nonwoven carrier which is impregnated with an impregnation medium, where both surfaces (sides) of the nonwoven carrier have a flat structure, the nonwoven carrier is provided with holes and is water-jet-consolidated, the impregnation medium is an oily, emulsion-like or surfactant-containing impregnation medium, **characterized in that** the number of holes per square centimetre is greater than/equal to 15, the fibres of the nonwoven carrier have a water absorption capacity of more than 5 g/g and the fibres of the nonwoven carrier have a water absorption rate of more than 60 mm/(10 min).

2. Skin cleansing towelette according to Claim 1, **characterized in that** the nonwoven carrier has a weight of 35-120 g/m².

3. Skin cleansing towelette according to one of Claims 1 or 2, **characterized in that** the nonwoven carrier is 0.4-2 mm thick.

4. Skin cleansing towelette according to one of Claims 1 - 3, **characterized in that** the holes are punched into the nonwoven carrier.

5. Skin cleansing towelette according to one of Claims 1 - 4, **characterized in that** the nonwoven carrier has a single-ply structure.

6. Skin cleansing towelette according to one of Claims 1 - 5, **characterized in that** the nonwoven carrier is produced on a flat surface.

7. Skin cleansing towelette according to one of Claims 1 - 6, **characterized in that** the impregnation medium constitutes 100 - 400% by weight, based on the weight of the nonwoven carrier.

8. Use of a skin cleansing towelette according to one of Claims 1 - 7 for skin-friendly, gentle and compatible skin cleansing, in particular for face cleansing, particularly for removing water-resistant make-up.

9. Process for producing a skin cleansing towelette according to one of Claims 1 - 7, **characterized in that** the nonwoven carrier is constructed and water-jet-consolidated on a smooth surface so that both of its sides have a smooth structure, then holes are punched into the nonwoven carrier produced in this way, the nonwoven carrier is impregnated with the impregnation medium and the product is, where necessary, cut to a suitable size and packaged.

## Revendications

1. Lingette de nettoyage de la peau avec un support en non-tissé, qui est imbibé d'un milieu d'imbibition, les deux surfaces (faces) du support en non-tissé étant planes, le support en non-tissé étant pourvu de trous et solidifié au jet d'eau, le milieu d'imbibition étant un milieu d'imbibition huileux, de type émulsion ou contenant un tensioactif, **caractérisée en ce que** le nombre de trous par centimètre carré est supérieur/égal à 15, les fibres du support en non-tissé présentent un pouvoir d'absorption de l'eau supérieur à 5 g/g et les fibres du support en non-tissé présentent une vitesse d'absorption d'eau supérieure à 60 mm/(10 min).

2. Lingette de nettoyage de la peau selon la revendication 1, **caractérisée en ce que** le support en non-tissé présente un poids surfacique de 35-120 g/m².

3. Lingette de nettoyage de la peau selon l'une quelconque des revendications 1 ou 2, **caractérisée en ce que** le support en non-tissé présente une épaisseur de 0,4-2 mm.

4. Lingette de nettoyage de la peau selon l'une quelconque des revendications 1-3, **caractérisée en ce que** les trous dans le support en non-tissé sont perforés.

5. Lingette de nettoyage de la peau selon l'une quelconque des revendications 1-4, **caractérisée en ce que** le support en non-tissé est formé avec une couche.

6. Lingette de nettoyage de la peau selon l'une quelconque des revendications 1-5, **caractérisée en ce que** le support en non-tissé est réalisé sur une surface plane.

7. Lingette de nettoyage de la peau selon l'une quelconque des revendications 1-6, **caractérisée en ce que** le milieu d'imbibition représente 100-400% en poids par rapport au poids du support en non-tissé.

8. Utilisation d'une lingette de nettoyage de la peau selon l'une quelconque des revendications 1-7 pour le nettoyage de la peau ménageant la peau, doux et toléré, en particulier pour le nettoyage du visage, en particulier pour l'élimination de maquillage résistant à l'eau.

9. Procédé de production d'une lingette de nettoyage de la peau selon l'une quelconque des revendications 1-7, **caractérisé en ce que** le support en non-tissé est élaboré sur une surface plane et solidifié par un jet d'eau de telle manière que ses deux faces sont formées en étant planes, puis on perfore des trous dans le support en non-tissé ainsi réalisé, le support en non-tissé est imbibé du milieu d'imbibition et le produit est le cas échéant découpé en une taille pratique et emballé.
